# EUROPEAN PATENT APPLICATION

(11) **EP 4 467 065 A1**
(43) Date of publication of application: **27.11.2024**
(21) Application number: 24177331.6
(22) Date of filing: 22.05.2024
(51) Int. Cl.: A61B 5/00, A61B 5/024, A61B 5/026, A61B 5/1455, A61B 5/16, A61B 5/117, A61B 5/18

(54) **ELECTRONIC DEVICE, PHYSIOLOGICAL STATE DETECTION DEVICE, AND PHYSIOLOGICAL STATE DETECTION METHOD APPLIED TO ELECTRONIC DEVICE**

(30) Priority: 25.05.2023 US 202363468797 P; 25.05.2023 US 202363468796 P; 25.05.2023 US 202363468798 P; 25.05.2023 US 202363468799 P
(71) Applicant: Deep Obverse Taiwan Inc., Taipei City R.O.C. 105412 (TW)
(72) Inventor: LIAO, CHIEN-SHOU, 105412 Taipei City (TW)
(74) Representative: karo IP

(57) **Abstract**

An electronic device, a physiological state detection device (D) and a physiological state detection method are provided. The electronic device configured for using the physiological state detection device (D) includes a device casing module, a signal control module (2), an image capturing module (3) and an information providing module (5). The image capturing module (3) and the information providing module (5) are disposed inside or outside the device casing module and electrically connected to the signal control module (2). When the image capturing module (3) is optionally configured to be used, the image capturing module (3) can be allowed to be configured through the signal control module (2) to continuously or discontinuously capture a plurality of eye images (M) of a user (U). When the information providing module (5) is optionally configured to be used, the information providing module (5) can be allowed to be configured through the signal control module (2) to present a physiological state signal (S2) corresponding to the eye images (M).

## Description

### FIELD OF THE INVENTION

The present invention relates to a physiological state detection device and a physiological state detection method, and more particularly to an electronic device configured for using the physiological state detection device, a physiological state detection device applied to the electronic device, and a physiological state detection method applied to the electronic device.

### BACKGROUND OF THE INVENTION

In the related art, a user can monitor the user's own health state and perform health management through physiological data and long-term records obtained by measuring hands through a physiological detection device. However, the physiological detection device in the related art still has room for improvement.

### SUMMARY OF THE INVENTION

In response to the above-referenced technical inadequacy, the present invention provides a physiological state detection device, an electronic device configured for using the physiological state detection device, and a physiological state detection method applied to the electronic device.

In order to solve the above-mentioned problems, one of the technical aspects adopted by the present invention is to provide a physiological state detection device applied to an electronic device, which includes a device casing module, a signal control module, an image capturing module, a wireless transmission module, an information providing module and a power supply module. The signal control module is disposed inside the device casing module. The image capturing module is electrically connected to the signal control module. The wireless transmission module is disposed inside the device casing module and electrically connected to the signal control module. The information providing module is disposed inside the device casing module and electrically connected to the signal control module. The power supply module is disposed inside the device casing module and electrically connected to the signal control module. When the image capturing module is optionally configured to be used, the image capturing module is allowed to be configured through the signal control module to continuously or discontinuously capture a plurality of eye images of a user using the electronic device within a predetermined period, thereby obtaining a plurality of eye image signals respectively corresponding to the eye images of the user. When the wireless transmission module is optionally configured to be used, the wireless transmission module is allowed to be configured through the signal control module to transmit the eye image signals to an information processing system, thereby obtaining a physiological state signal corresponding to the eye image signals. When the wireless transmission module is optionally configured to be used, the wireless transmission module is allowed to be configured through the signal control module to receive the physiological state signal that is obtained through processing by the information processing system. When the information providing module is optionally configured to be used, the information providing module is allowed to be configured through the signal control module to present the physiological state signal for reference by relevant personnel. When the power supply module is optionally configured to be used, the power supply module is allowed to be configured through the signal control module to supply power to the signal control module, the image capturing module, the wireless transmission module and the information providing module. Each of the eye images of the user includes at least one scleral image with microvascular characteristics or at least one eyelid image with microvascular characteristics.

In order to solve the above-mentioned problems, another one of the technical aspects adopted by the present invention is to provide an electronic device configured for using a physiological state detection device, which includes a device casing module, a signal control module, an image capturing module and an information providing module. The signal control module is disposed inside the device casing module. The image capturing module is electrically connected to the signal control module. The information providing module is disposed inside the device casing module and electrically connected to the signal control module. When the image capturing module is optionally configured to be used, the image capturing module is allowed to be configured through the signal control module to continuously or discontinuously capture a plurality of eye images of a user. When the information providing module is optionally configured to be used, the information providing module is allowed to be configured through the signal control module to present a physiological state signal corresponding to the eye images. Each of the eye images of the user includes at least one scleral image with microvascular characteristics or at least one eyelid image with microvascular characteristics.

In order to solve the above-mentioned problems, yet another one of the technical aspects adopted by the present invention is to provide a physiological state detection method, which includes providing an electronic device equipped with a physiological state detection device; identifying an identity of a user using the electronic device through a biometric module of the physiological state detection device; continuously or discontinuously capturing a plurality of eye images of the user within a predetermined period by an image capturing module of the physiological state detection device, thereby obtaining blood flow changes or spectral changes in capillaries of the user's scleras or eyelids; processing the eye images by an information processing system, thereby obtaining a physiological state signal corresponding to the eye images; and presenting the physiological state signal by an information providing module of the physiological state detection device for reference by relevant personnel. When the information providing module is allowed to be configured as an information display for displaying the physiological state signal, the information display is allowed to be configured to visually present the physiological state signal. When the information providing module is allowed to be configured as an information projector for projecting the physiological state signal to at least one eye based on eyeball position information captured by an eye tracking module, the information projector is allowed to be configured to visually present the physiological state signal. When the information providing module is allowed to be configured as a sound player for playing the physiological state signal, the sound player is allowed to be configured to audibly present the physiological state signal. When the information providing module is allowed to be configured as a vibration generator for generating different vibration frequencies based on changes in the physiological state signal, the vibration generator is configured to tangibly present the physiological state signal.

Therefore, in the physiological state detection device and the electronic device provided by the present invention, by virtue of "the image capturing module being electrically connected to the signal control module" and "the information providing module being disposed inside the device casing module and electrically connected to the signal control module," when the image capturing module is optionally configured to be used, the image capturing module can be allowed to be configured through the signal control module to continuously or discontinuously capture a plurality of eye images (each eye image includes at least one scleral image with microvascular characteristics or at least one eyelid image with microvascular characteristics) or a plurality of facial images (each facial image includes at least one scleral image with microvascular characteristics or at least one eyelid image with microvascular characteristics) of a user, and when the information providing module is optionally configured to be used, the information providing module can be allowed to be configured through the signal control module to present a physiological state signal corresponding to the eye images for reference by relevant personnel.

Furthermore, in the physiological state detection method provided by the present invention, by virtue of "providing an electronic device equipped with a physiological state detection device," "identifying an identity of a user using the electronic device through a biometric module of the physiological state detection device," "continuously or discontinuously capturing a plurality of eye images of the user within a predetermined period by an image capturing module of the physiological state detection device, thereby obtaining blood flow changes or spectral changes in capillaries of the user's scleras or eyelids" and "processing the eye images by an information processing system, thereby obtaining a physiological state signal corresponding to the eye images," the physiological state signal can be presented by an information providing module of the physiological state detection device for reference by relevant personnel. For example, when the information providing module is allowed to be configured as an information display for displaying the physiological state signal, the information display can be allowed to be configured to visually present the physiological state signal for reference by relevant personnel. When the information providing module is allowed to be configured as an information projector for projecting the physiological state signal to at least one eye based on eyeball position information captured by an eye tracking module, the information projector can be allowed to be configured to visually present the physiological state signal for reference by relevant personnel. When the information providing module is allowed to be configured as a sound player for playing the physiological state signal, the sound player can be allowed to be configured to audibly present the physiological state signal for reference by relevant personnel. When the information providing module is allowed to be configured as a vibration generator for generating different vibration frequencies based on changes in the physiological state signal, the vibration generator can be configured to tangibly present the physiological state signal for reference by relevant personnel.

These and other aspects of the present invention will become apparent from the following description of the embodiment taken in conjunction with the following drawings and their captions, although variations and modifications therein may be affected without departing from the spirit and scope of the novel concepts of the invention.

### BRIEF DESCRIPTION OF THE DRAWINGS

The described embodiments may be better understood by reference to the following description and the accompanying drawings, in which:
FIG. 1 is a functional block diagram of a physiological state detection device provided by a first embodiment of the present invention;
FIG. 2 is a schematic perspective view of an electronic device using the physiological state detection device provided by the present invention;
FIG. 3 is another schematic perspective view of the electronic device using the physiological state detection device provided by the present invention;
FIG. 4 is a schematic view of a pair of eyeglasses using the physiological state detection device being worn by a user according to the present invention;
FIG. 5 is a schematic view of the user's eye image captured through an image capturing module provided by the eyeglasses (or a helmet) using the physiological state detection device provided by the present invention;
FIG. 6 is a schematic view of the helmet using the physiological state detection device being worn by the user according to the present invention;
FIG. 7 is a schematic view of an eye mask using the physiological state detection device being worn by the user according to the present invention;
FIG. 8 is a schematic view of the user's eyelid image captured through the image capturing module provided by the eye mask using the physiological state detection device provided by the present invention;
FIG. 9 is a flowchart of the physiological state detection method provided by the first embodiment of the present invention; and
FIG. 10 is a functional block diagram of the physiological state detection device provided by a second embodiment of the present invention.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

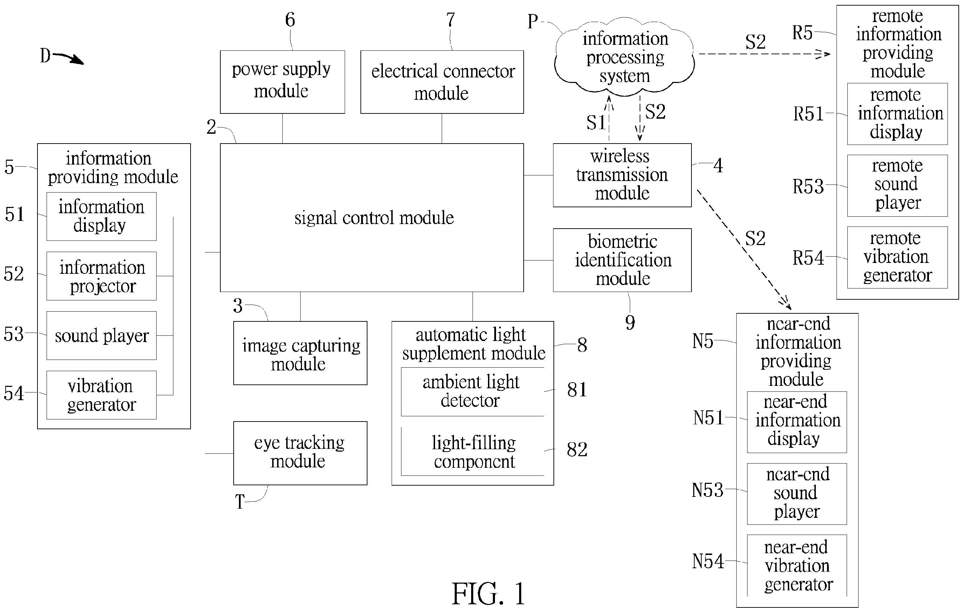

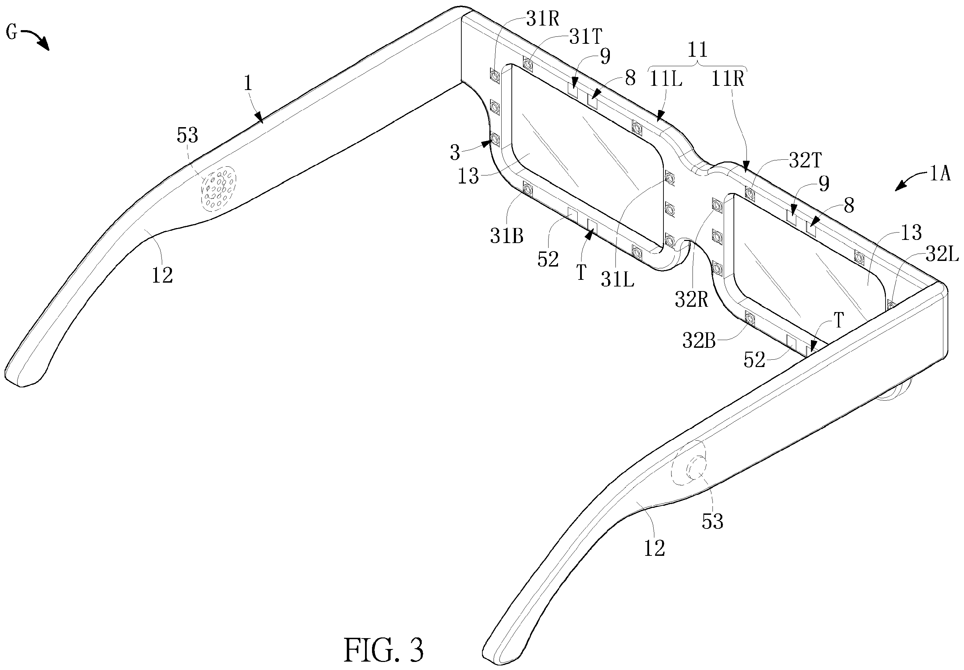

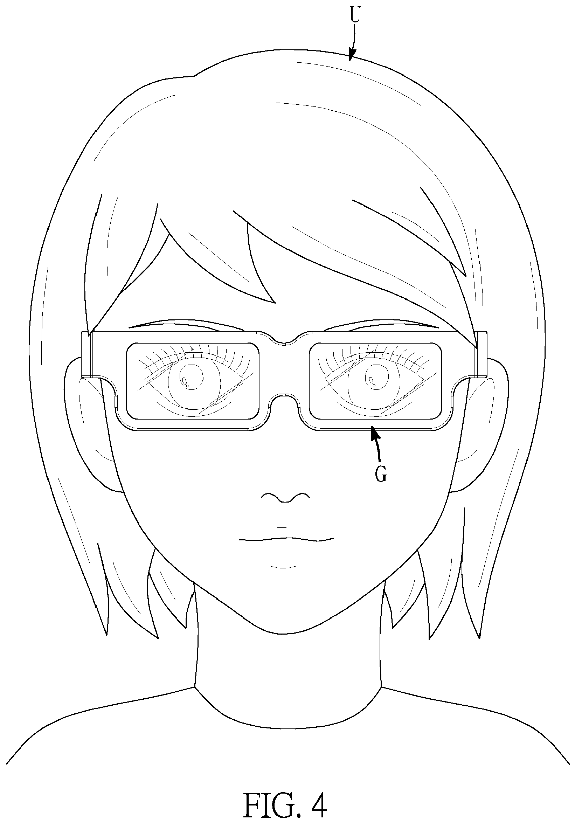

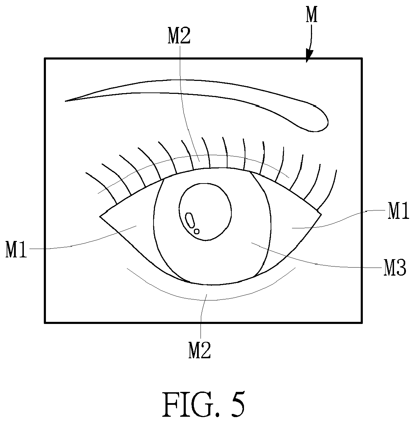

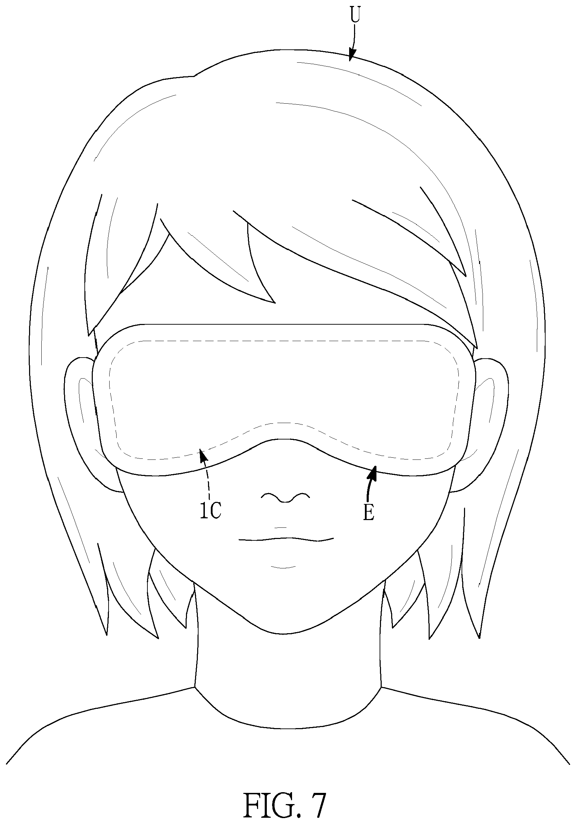

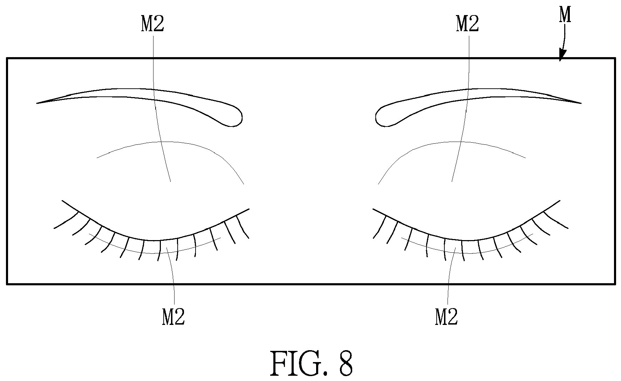

Referring to FIG. 1 to FIG. 10, the present invention provides a physiological state detection device D applied to an electronic device (such as a head-mounted device configured as a pair of smart eyeglasses G, a smart helmet H, or a smart eye mask E, or such as a wearable device configured as a smart contact lens, a smart ring, a smart band, a smart watch or a smart bracelet, or such as a desktop computer, a portable computer, a card reader of an access control system, a transportation or any kind of vehicle such as the steering wheel of the vehicle, which can be configured for using the physiological state detection device D), which includes a device casing module (such as an eyeglass structure module 1A, a helmet structure module 1B, an eye mask structure module 1C), a signal control module 2, an image capturing module 3 and an information providing module 5 (that is to say, a wireless transmission module 4, an electrical connector module 7, an automatic light supplement module 8 and a biometric module 9 (or a biometric recognition module) can be omitted in the physiological state detection device D according to different requirements). More particularly, the signal control module 2 can be disposed inside the device casing module, the image capturing module 3 can be disposed inside the device casing module (or outside the device casing module) and electrically connected to the signal control module 2, and the information providing module 5 can be disposed inside the device casing module and electrically connected to the signal control module 2. Therefore, when the image capturing module 3 is optionally configured to be used, the image capturing module 3 can be allowed to be configured through the signal control module 2 to continuously or discontinuously capture a plurality of eye images M (as shown in FIG. 5) of a user U using the electronic device within a predetermined period (for example, the eye images M can be captured from one or both eyes of the user U according to different requirements), or continuously or discontinuously capture a plurality of facial images of a user U using the electronic device within a predetermined period. In addition, when the information providing module 5 is optionally configured to be used, the information providing module 5 can be allowed to be configured through the signal control module 2 to present a physiological state signal S2 corresponding to the eye images M for reference by relevant personnel. It should be noted that each of the eye images M of the user U includes at least one scleral image M1 with microvascular characteristics or at least one eyelid image M2 with microvascular characteristics (or each of the facial images of the user U includes at least one scleral image M1 with microvascular characteristics or at least one eyelid image M2 with microvascular characteristics), so that blood flow changes (such as changes in blood flow velocity or blood flow conditions of the microvessels between the scleral images M1 or between the eyelid images M2) or spectral changes (such as absorption spectrum changes, emission spectrum changes, scattering spectrum changes or any kind of spectral changes) in the capillaries of the user U in the scleras or eyelids can be obtained by the image capturing module 3 of the physiological state detection device D provided by the present invention.

### [First Embodiment]

Referring to FIG. 1 to FIG. 8, a first embodiment of the present invention provides a physiological state detection device D (or a physiological and mental state detection device, or a physiological information detection device, or a physiological and mental information detection device) that can be applied to a pair of eyeglasses G, which includes an eyeglass structure module 1A, a signal control module 2, an image capturing module 3, a wireless transmission module 4, an information providing module 5 and a power supply module 6. For example, the physiological state detection device D can be applied to the electronic device or any electronic device similar to the eyeglasses G (such as a goggle worn by the user when exercising or working, a swimming goggle, a snow goggle, a pilot goggle, a firefighter goggle, a military goggle or a medical goggle) through an external contact method (or an insertion method), a built-in method (or an embedded method), or any setting method. However, the aforementioned details are disclosed for exemplary purposes only.

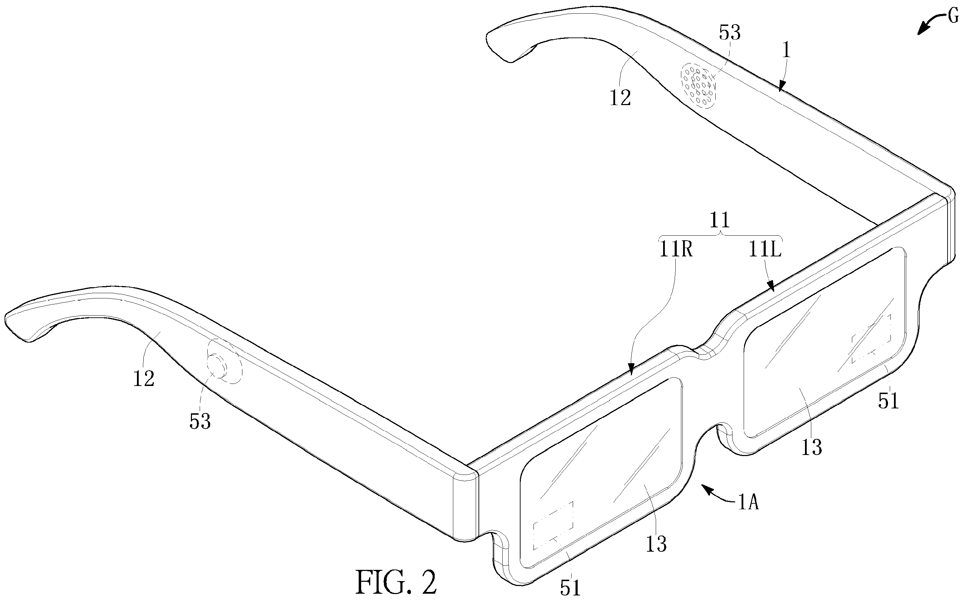

More particularly, referring to FIG. 2, FIG. 3 and FIG. 4, the eyeglass structure module 1A can be configured as the main external structure of the electronic device, or the eyeglass structure module 1A can be configured to cooperate with the eyeglasses G. For example, the eyeglass structure module 1A includes an eyeglass frame 11, two eyeglass temples 12 (two eyeglass legs) connected to the eyeglass frame 11, and two eyeglass lenses 13 (such as lenses with or without prescription, lenses with or without an anti-UV layer, lenses with or without anti-reflective layer, lenses with or without polarizing coating layer, or lenses with or without tinted coating layer to control or limit external ambient light entering the eyeball of the user U, or lenses with any electric-controlled coating layer which can block or limit the external light entering the eyeball of the user U) held or carried by the eyeglass frame 11. In one possible embodiment, the signal control module 2, the wireless transmission module 4, the power supply module 6 and the electrical connector module 7 can be disposed inside at least one of the two eyeglass temples 12 of the eyeglass structure module 1A, or disposed on any position inside the eyeglass structure module 1A in an embedded manner. Moreover, the image capturing module 3, the automatic light supplement module 8 and the biometric module 9 can be disposed inside the eyeglass frame 11 of the eyeglass structure module 1A, or disposed on any position inside the eyeglass structure module 1A in an embedded manner. In addition, the information providing module 5 can be disposed inside at least one of the eyeglass frame 11, the eyeglass temple 12 and the eyeglass lens 13 of the eyeglass structure module 1A, or disposed on any position inside the eyeglass structure module 1A in an embedded manner). However, the aforementioned details are disclosed for exemplary purposes only.

More particularly, referring to FIG. 1 to FIG. 5, the signal control module 2 can be disposed inside the eyeglass structure module 1A, and the image capturing module 3 can be disposed inside the eyeglass structure module 1A (or a part of the image capturing module 3 can be exposed outside the eyeglass structure module 1A) and electrically connected to the signal control module 2. For example, the image capturing module 3 includes a first left image capturing group 31L (including multiple first left image sensors) and a first right image capturing group 31R (including multiple first right image sensors) corresponding to the first left image capturing group 31L, and the first left image capturing group 31L and the first right image capturing group 31R can be disposed on a left side and a right side of a left frame 11L of the eyeglass frame 11, respectively. Moreover, the image capturing module 3 includes a first upper image capturing group 31T (including multiple first upper image sensors) and a first lower image capturing group 31B (including multiple first lower image sensors) corresponding to the first upper image capturing group 31T, and the first upper image capturing group 31T and the first lower image capturing group 31B can be disposed on an upper side and a lower side of the left frame 11L of the eyeglass frame 11, respectively. Moreover, the image capturing module 3 includes a second left image capturing group 32L (including multiple second left image sensors) and a second right image capturing group 32R (including multiple second right image sensors) corresponding to the second left image capturing group 32L, and the second left image capturing group 32L and the second right image capturing group 32R can be disposed on a left side and a right side of a right frame 11R of the eyeglass frame 11, respectively. In addition, the image capturing module 3 includes a second upper image capturing group 32T (including multiple second upper image sensors) and a second lower image capturing group 32B (including multiple second lower image sensors) corresponding to the second upper image capturing group 32T, and the second upper image capturing group 32T and the second lower image capturing group 32B can be disposed on an upper side and a lower side of the right frame 11R of the eyeglass frame 11, respectively. However, the aforementioned details are disclosed for exemplary purposes only.

Based on the above, referring to FIG. 1 to FIG. 5, when the image capturing module 3 is optionally configured to be used, the image capturing module 3 can be allowed to be configured through the signal control module 2 to continuously (or continuously and regularly) or discontinuously (or non-continuously and regularly) capture a plurality of eye images M (or eye images and characteristic images around the eyes) of a user U using (or wearing) the electronic device within a predetermined period (such as within tens of seconds or minutes), thereby obtaining a plurality of eye image signals S 1 (or signals of eye images and characteristic images around the eyes) respectively corresponding to the eye images M of the user U, and each of the eye images M of the user U includes at least one or more scleral images M1 (or images of white of the eye) with microvascular characteristics (such as capillaries in the scleral) or at least one or more eyelid images M2 (or eyelid area images each including an upper eyelid image and a lower eyelid image) with microvascular characteristics (such as capillaries in the skin). In addition, the number of the eye images M or the facial images obtained by the image capturing module 3 within the predetermined period can exceed a predetermined value (for example, the number of the eye images M or the facial images can be any positive integer between 10 and 200, or any positive integer exceeding 200), thereby obtaining blood flow changes (such as changes in blood flow velocity or blood flow conditions of the microvessels or capillaries between the scleral images M1 or between the eyelid images M2) or spectral changes (such as the spectral changes or dark line spectral changes in the microvessels or capillaries between the scleral images M1 or between the eyelid images M2, for example, spectral changes may include absorption spectrum changes, emission spectrum changes, scattering spectrum changes or any kind of spectral changes) in capillaries of scleras or eyelids of the user U. More particularly, the image capturing module 3 includes at least one lower pixel facial image capturer (such as a low-level pixel image sensor that can be configured to roughly find the eye position of the user U so as to obtain a rough image such as an eye contour image or a facial contour image) and at least one higher pixel eye image capturer (such as a high-level pixel image sensor that can be configured to clearly and correctly obtain an eye image of the user U), and the at least one lower pixel facial image capturer can be configured to confirm an eye position range (such as including a facial contour and an eye contour) of the user U to obtain eye position information, and the at least one higher pixel eye image capturer can be configured to quickly and accurately capture the at least one scleral image M1 or the at least one eyelid image M2 of the user U based on the eye position information provided by the at least one lower pixel facial image capturer. In addition, the image capturing module 3 includes an angle-adjustable lens structure (such as an angle adjustment lens structure that can be adjusted arbitrarily within a predetermined angle range), and the angle-adjustable lens structure can be configured to manually or automatically adjust an image capturing angle relative to the user U. Therefore, when the image capturing module 3 is optionally configured to be used, the image capturing module 3 can be allowed to be configured through the signal control module 2 to manually or automatically (or continuously or discontinuously) adjust the image capturing angle of the angle-adjustable lens structure relative to the user U, until the facial image M of the user U is completely captured by the image capturing module 3. It should be noted that the signal control module 2 can use a central processing unit (CPU), a digital signal processor (DSP), a microprocessor (MPU), a microcontroller (MCU) or any type of control chip with any type of memory. In addition, the image capturing module 3 may include one or more image sensors or image readers, the image capturing module 3 may also include multiple image sensors or image readers of the same type or different types, and the image sensor used in the image capturing module 3 can be a charge-coupled device (CCD) image sensor, a complementary metal oxide semiconductor (CMOS) image sensor, or any type of image sensor. However, the aforementioned details are disclosed for exemplary purposes only.

More particularly, referring to FIG. 1 and FIG. 2, the wireless transmission module 4 can be disposed inside the eyeglass structure module 1A and electrically connected to the signal control module 2. For example, when the wireless transmission module 4 is optionally configured to be used, the wireless transmission module 4 can be allowed to be configured through the signal control module 2 to wirelessly transmit the eye image signals S1 to an information processing system P (that is to say, the physiological state detection device D and the information processing system P can communicate with each other in a wireless manner), thereby obtaining a physiological state signal S2 (or physiological and mental state signals, or physical and mental state signals, which may include, for example, heart rate, blood pressure, blood oxygen, lactate, blood sugar, sleepiness and alcohol concentration, or any kind of physiological and mental information) corresponding to the eye image signals S 1. In one possible embodiment, physiological information may include, for example, physical and mental reference indexes (such as physiological stress index, depression index, fatigue level, etc.), physiological monitoring values (such as heart rate, blood pressure, blood oxygen, lactate, blood sugar, etc.), and disease risk assessment factors (such as high blood pressure, heart disease, myocardial infarction, diabetes, Alzheimer's disease, Parkinson's disease, cancer, stroke probability, etc.). In addition, when the wireless transmission module 4 is optionally configured to be used, the wireless transmission module 4 can be allowed to be configured through the signal control module 2 to wirelessly receive the physiological state signal S2 that is obtained through processing by the information processing system P. More particularly, the wireless transmission module 4 can perform wireless data transmission through the cooperation of the antenna structure (or antenna chip) with Wi-Fi, Bluetooth, ZigBee or any wireless transmission method. Moreover, the information processing system P can be configured anywhere (such as a data processing center) away from the electronic device or can be installed directly inside the electronic device, the information processing system P at least includes a database and an information processing device, and the information processing device can be configured to compare and calculate the eye image signals S1 based on the database (or the big data), thereby obtaining the physiological state signal S2 (such as heart rate, blood pressure, blood oxygen, lactate, blood sugar, sleepiness and alcohol concentration). However, the aforementioned details are disclosed for exemplary purposes only.

More particularly, referring to FIG. 1, FIG. 2, FIG. 3 and FIG. 4, the information providing module 5 can be disposed inside the eyeglass structure module 1A (or a part of the information providing module 5 can be exposed outside the eyeglass structure module 1A) and electrically connected to the signal control module 2. For example, when the information providing module 5 is optionally configured to be used, the information providing module 5 can be allowed to be configured through the signal control module 2 to present the physiological state signal S2 for reference by relevant personnel (such as the user U using the electronic device, the companions close to the user U, the remote controller of the electronic device, or the family members or the doctors away from the electronic device). In addition, the information providing module 5 can be allowed to be configured as an information display 51 (such as a transparent display) for visually presenting the physiological state signal S, an information projector 52 for visually presenting the physiological state signal S, a sound player 53 for audibly presenting the physiological state signal S2 and a vibration generator 54 for tangibly presenting the physiological state signal S2, or at least one or more of the information display 51, the information projector 52, the sound player 53 and the vibration generator 54 can cooperate with each other to present the physiological state signal S2. However, the aforementioned details are disclosed for exemplary purposes only.

For example, referring to FIG. 1, FIG. 2, FIG. 3 and FIG. 4, when the information providing module 5 is optionally configured to be used, the information providing module 5 can be allowed to be configured as at least one or more information displays 51 (such as data displays that can display different data, or light displays that can display lights of different colors) for displaying the physiological state signal S2, so that the information display 51 can be allowed to be configured to present (or display) the physiological state signal S2 (such as image signals) in a visible or visual manner (such as displaying numbers, text, images or colors) through the control of the signal control module 2 for reference by relevant personnel (for example, as shown in FIG. 2, the physiological state signal S2 can be presented or displayed to others for reference in an obvious and easily visible manner or in an externally displaying manner through the information display 51 that is provided at any position of the eyeglass lens 13 of the eyeglass structure module 1A or at any position of the eyeglass structure module 1A). Furthermore, when the information providing module 5 is optionally configured to be used, the information providing module 5 can be allowed to be configured as at least one or more information projectors 52 (such as small projectors) for projecting the physiological state signal S2 to at least one or two eyes based on or according to eyeball position information (or at least one or more eyeball position signals) captured by at least one or more eye tracking module T, so that the information projector 52 can be allowed to be configured to present (or display) the physiological state signal S2 (such as image signals) in a visible or visual manner (such as displaying numbers, text, images or colors) through the control of the signal control module 2 for reference by relevant personnel. Moreover, when the information providing module 5 is optionally configured to be used, the information providing module 5 can be allowed to be configured as at least one or more sound players 53 (such as small speakers) for playing the physiological state signal S2, so that the sound player 53 can be allowed to be configured to present (or play) the physiological state signal S2 (such as audio signals) in an audible or auditory manner (such as playing pure tones or polyphonic tones, or musical tones or non-musical tones) through the control of the signal control module 2 for reference by relevant personnel. In addition, when the information providing module 5 is optionally configured to be used, the information providing module 5 can be allowed to be configured as at least one or more vibration generators 54 (such as small vibration motors) for generating different vibration frequencies based on or according to changes in the physiological state signal S2, so that the vibration generator 54 can be configured to present (or generate) the physiological state signal S2 (such as vibration signals) in a touchable or tactile manner (such as generating continuous or discontinuous vibrations, or generating high-frequency or low-frequency vibrations) through the control of the signal control module 2 for reference by relevant personnel. However, the aforementioned details are disclosed for exemplary purposes only.

For example, referring to FIG. 1 and FIG. 2, when the wireless transmission module 4 is optionally configured to be used, the wireless transmission module 4 can be allowed to be configured through the signal control module 2 to wirelessly transmit the received physiological state signal S2 to a near-end information providing module N5 (such as a proximal information providing module provided by the portable device of the user U) adjacent to the user U, and the physiological state detection device D can be configured to perform information acquisition operation (such as capturing image information), information transmission operation (such as transmitting image signal), and information calculation operation (such as calculating image signal) through the control of the near-end information providing module N5, or the physiological state detection device D can also perform any operation that can issue any command to the physiological state detection device D. More particularly, when the near-end information providing module N5 is optionally configured to be used, the near-end information providing module N5 can be allowed to be configured as at least one or more near-end information displays N51 (such as an image display provided by the user's portable device, e.g., mobile phone or tablet) for displaying the physiological state signal S2, so that the near-end information display N51 can be allowed to be configured to present (or display) the physiological state signal S2 in a visible or visual manner for reference by relevant personnel. Moreover, when the near-end information providing module N5 is optionally configured to be used, the near-end information providing module N5 can be allowed to be configured as at least one or more near-end sound players N53 (such as a speaker provided by the user's portable device, e.g., a mobile phone, tablet or wireless headphone) for playing the physiological state signal S2, so that the near-end sound player N53 can be allowed to be configured to present (or play) the physiological state signal S2 in an audible or auditory manner for reference by relevant personnel. In addition, when the near-end information providing module N5 is optionally configured to be used, the near-end information providing module N5 can be allowed to be configured as at least one or more near-end vibration generators N54 (such as a vibration motor provided by the user's portable device, e.g., a mobile phone, tablet or wireless headphone) for generating different vibration frequencies based on or according to changes in the physiological state signal S2, so that the near-end vibration generator N54 can be allowed to be configured to present (or generate) the physiological state signal S2 in a touchable or tactile manner for reference by relevant personnel. However, the aforementioned details are disclosed for exemplary purposes only.

For example, as shown in FIG. 1, when the information processing system P can be configured to wirelessly transmit the processed physiological state signal S2 to a remote information providing module R5 (for example, it can be a portable electronic device such as a mobile phone, a tablet or a laptop, or a non-portable electronic device such as a desktop computer or a large system computer) away from the user U, the remote information providing module R5 can be allowed to be configured as at least one or more remote information displays R51 (such as any kind of display) for displaying the physiological state signal S2, so that the remote information display R51 can be allowed to be configured to present (or display) the physiological state signal S2 in a visible or visual manner for reference by relevant personnel. Alternatively, when the information processing system P can be configured to wirelessly transmit the physiological state signal S2 to a remote information providing module R5 away from the user U, the remote information providing module R5 can be allowed to be configured as at least one or more remote sound players R53 (such as any kind of speaker) for playing the physiological state signal S2, so that the remote sound player R53 can be allowed to be configured to present (or play) the physiological state signal S2 in an audible or auditory manner for reference by relevant personnel. Alternatively, when the information processing system P can be configured to wirelessly transmit the physiological state signal S2 to a remote information providing module R5 away from the user U, the remote information providing module R5 can be allowed to be configured as at least one or more remote vibration generators R54 (such as any kind of vibration motor) for generating different vibration frequencies based on or according to changes in the physiological state signal S2, so that the remote vibration generator R54 can be allowed to be configured to present (or generate) the physiological state signal S2 in a touchable or tactile manner for reference by relevant personnel. However, the aforementioned details are disclosed for exemplary purposes only.

More particularly, as shown in FIG. 1, the power supply module 6 can be disposed inside the eyeglass structure module 1A and electrically connected to the signal control module 2. For example, when the power supply module 6 is optionally configured to be used, the power supply module 6 can be allowed to be configured through the signal control module 2 to supply power to the signal control module 2, the image capturing module 3, the wireless transmission module 4 and the information providing module 5. In addition, the power supply module 6 can be any kind of rechargeable battery or solar cell. However, the aforementioned details are disclosed for exemplary purposes only.

It should be noted that, for example, as shown in FIG. 1, the physiological state detection device D further includes an electrical connector module 7 disposed inside the eyeglass structure module 1A (or a part of the electrical connector module 7 can be exposed outside the eyeglass structure module 1A) and electrically connected to the signal control module 2. More particularly, when the electrical connector module 7 is optionally configured to be used (such as using USB or any kind of transmission interface), the electrical connector module 7 can be allowed to be configured through the signal control module 2 to communicate with an external system in a wired manner (such as transmitting data or current). However, the aforementioned details are disclosed for exemplary purposes only.

It should be noted that, for example, as shown in FIG. 1, the physiological state detection device D further includes an automatic light supplement module 8 (or an automatic light fill module) disposed inside the eyeglass structure module 1A (or a part of the automatic light supplement module 8 can be exposed outside the eyeglass structure module 1A) and electrically connected to the signal control module 2. More particularly, the automatic light supplement module 8 includes an ambient light detector 81 and a light-filling component 82, and the light-filling component 82 can be a fill light for generating invisible light or visible light (or can be an IR LED fill light or an RGB LED fill light that can use any kind of LED, or can be a fill light that can use any kind of light-emitting element). Therefore, when the ambient light detector 81 is optionally configured to be used, the ambient light detector 81 can be allowed to be configured through the signal control module 2 to detect an ambient light around the user U, thereby obtaining ambient light information. In addition, when the light-filling component 82 is optionally configured to be used, the light-filling component 82 can be allowed to be configured through the signal control module 2 to determine whether to provide a predetermined invisible light (or a predetermined visible light) to the eyes of the user U based on whether the ambient light information (or ambient light conditions) meets a predetermined requirement, thereby improving the effect of the image capturing module 3 for capturing the eye images M (such as improving the contrast, brightness and clarity of the image). However, the aforementioned details are disclosed for exemplary purposes only.

It should be noted that, for example, as shown in FIG. 1, the physiological state detection device D further includes a biometric module 9 disposed inside the eyeglass structure module 1A (or a part of the biometric module 9 can be exposed outside the eyeglass structure module 1A) and electrically connected to the signal control module 2. More particularly, the biometric module 9 can be configured as an iris recognition module (or an iris identification module), a sclera recognition module (or a sclera identification module, such as for recognizing or identifying sclera blood vessels), a palmprint recognition module (or a palmprint identification module), a fingerprint recognition module (or a fingerprint identification module), a facial recognition module (or a facial identification module) or any kind of biometric module, or the biometric module 9 can also integrate the iris recognition module, the sclera recognition module, the palmprint recognition module, the fingerprint recognition module, the facial recognition module and any kind of biometric module at the same time. Therefore, when the biometric module 9 can be configured as the iris recognition module or the sclera recognition module, the iris recognition module or the sclera recognition module can be allowed to be configured through the signal control module 2 to capture at least one or more iris images M3 or sclera blood vessel images of the user U (i.e., the iris image M3 or the sclera blood vessel image provided from the eye image M of the user U), thereby identifying or determining (or confirming the identity of the user U to determine) whether the user U is qualified to use the physiological state detection device D. In addition, when the biometric module 9 can be configured as the fingerprint recognition module or the palmprint recognition module, the fingerprint recognition module or the palmprint recognition module can be allowed to be configured through the signal control module 2 to capture at least one or more fingerprint images or palmprint images of the user U (i.e., the fingerprint image or the palmprint image provided from the finger of the user U), thereby identifying or determining (or confirming the identity of the user U to determine) whether the user U is qualified to use the physiological state detection device D. However, the aforementioned details are disclosed for exemplary purposes only.

It should be noted that, for example, depending on different requirements, the information projector 52 can be configured to project the physiological state signal S2 (in the form of an image beam) directly to the eyes of the user U, or the information projector 52 can be configured to indirectly project the physiological state signal S2 (in the form of an image beam) to the eyes of the user U through transmission (or through multiple reflections) inside the eyeglass lenses 13. However, the aforementioned details are disclosed for exemplary purposes only.

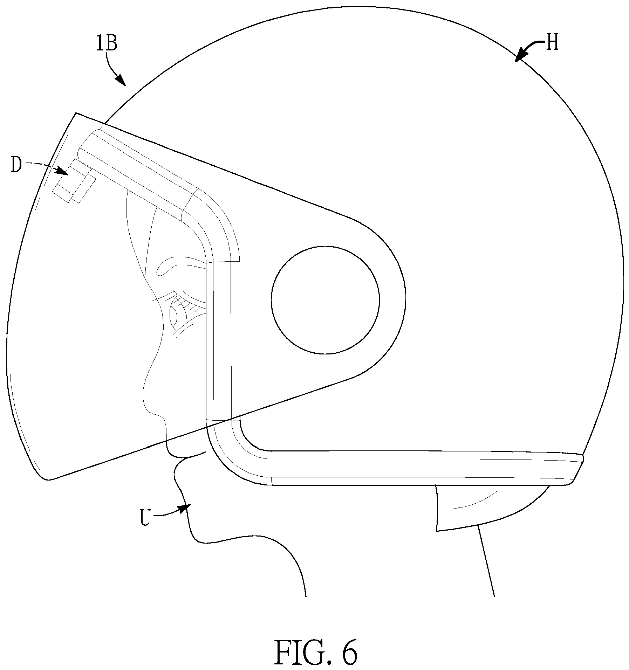

It should be noted that, for example, referring to FIG. 1, FIG. 5 and FIG. 6, the physiological state detection device D can also be applied to the helmet H. When the image capturing module 3 is optionally configured to be used, the image capturing module 3 can be allowed to be configured through the signal control module 2 to continuously or discontinuously capture a plurality of eye images M (each eye image M includes at least one scleral image M1 with microvascular characteristics or at least one eyelid image M2 with microvascular characteristics) or a plurality of facial images (each facial image includes at least one scleral image M1 with microvascular characteristics or at least one eyelid image M2 with microvascular characteristics) of the user U using (or wearing) the helmet H within a predetermined period. In addition, when the information providing module 5 is optionally configured to be used, the information providing module 5 can be allowed to be configured through the signal control module 2 to present a physiological state signal S2 corresponding to the eye images M for reference by relevant personnel (for example, the physiological state signal S2 can be presented or displayed to others for reference in an obvious and easily visible manner or in an externally displaying manner through the information display 51 that is provided at any position of the helmet lens of the helmet structure module 1B or at any position of the helmet structure module 1B). More particularly, when the physiological state detection device D is applied to the helmet H, the physiological state detection device D can be directly built into the helmet H (for example, the image capturing module 3, the automatic light supplement module 8 and the biometric module 9 can be disposed inside at least one of the helmet structure and the helmet lens of the helmet structure module 1B) or installed on the helmet H through an external contact method (or an insertion method as shown in FIG. 6).

It should be noted that, for example, referring to FIG. 1, FIG. 7 and FIG. 8, the physiological state detection device D can also be applied to the eye mask E. When the image capturing module 3 is optionally configured to be used, the image capturing module 3 can be allowed to be configured through the signal control module 2 to continuously or discontinuously capture a plurality of eye images M of the user U using (or wearing) the eye mask E within a predetermined period. More particularly, when the user's eyes are closed, each eye image M may include at least one eyelid image M2 or an eyelid area (including an upper eyelid and a lower eyelid, in which the upper eyelid can be completely exposed without wrinkles) with microvascular characteristics, thereby improving the image sharpness of capturing the capillary features of the upper eyelid area, such as edge sharpness (or acutance) and resolution. In addition, when the information providing module 5 is optionally configured to be used, the information providing module 5 can be allowed to be configured through the signal control module 2 to present a physiological state signal S2 corresponding to the eye images M for reference by relevant personnel (for example, the physiological state signal S2 can be presented or displayed to others for reference in an obvious and easily visible manner or in an externally displaying manner through the information display 51 that is provided at any position of the helmet lens of the eye mask structure module 1C or at any position of the eye mask structure module 1C).

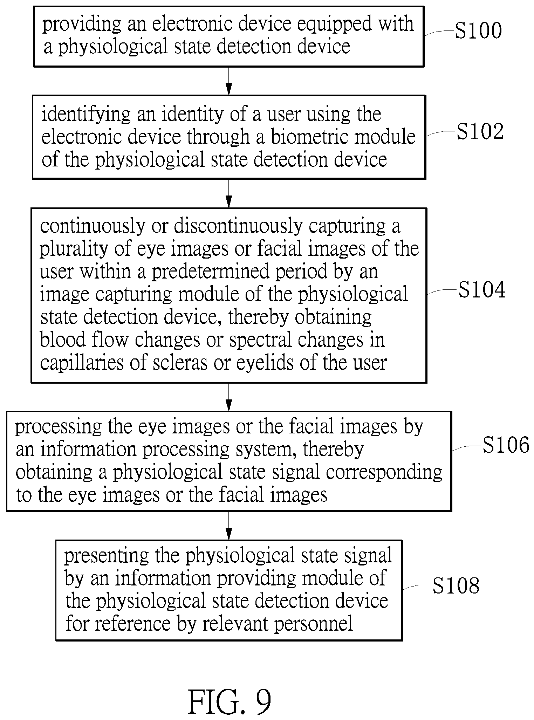

It should be noted that, referring to FIG. 1 to FIG. 9, the first embodiment of the present invention further provides a physiological state detection method applied to an electronic device, which includes: firstly, referring to FIG. 1, FIG. 4, FIG. 6, FIG. 7 and FIG. 9, providing an electronic device (such as a pair of eyeglasses G, a helmet H or an eye mask E) equipped with (or including) a physiological state detection device D (step S100); next, referring to FIG. 1, FIG. 4, FIG. 6, FIG. 7 and FIG. 9, identifying (or recognizing) an identity of a user U using (or wearing) the electronic device through a biometric module 9 of the physiological state detection device D (step S102); then, referring to FIG. 1, FIG. 5, FIG. 8 and FIG. 9, continuously or discontinuously capturing a plurality of eye images M of the user U within a predetermined period by an image capturing module 3 of the physiological state detection device D, thereby obtaining blood flow changes (such as changes in blood flow velocity or blood flow conditions of the microvessels or capillaries between the scleral images M1 or between the eyelid images M2) or spectral changes (such as absorption spectrum changes, emission spectrum changes, scattering spectrum changes or any kind of spectral changes) in capillaries of scleras or eyelids of the user U, or thereby obtaining a plurality of eye image signals S1 respectively corresponding to the eye images M of the user U (step S104); afterward, referring to FIG. 1 and FIG. 9, processing the eye images M (or processing the eye image signals S1) by an information processing system P, thereby obtaining a physiological state signal S2 corresponding to the eye images M (step S106); and then referring to FIG. 1, FIG. 2, FIG. 4 and FIG. 9, presenting (such as displaying, playing, generating) the physiological state signal S2 by an information providing module 5 of the physiological state detection device D for reference by relevant personnel (step S108). For example, when the information providing module 5 can be allowed to be configured as at least one or more information displays 51 for displaying the physiological state signal S2, the information display 51 can be allowed to be configured to visually present the physiological state signal S2. When the information providing module 5 can be allowed to be configured as at least one or more information projectors 52 for projecting the physiological state signal S2 to at least one or more eyes based on eyeball position information captured by at least one or more eye tracking modules T, the information projector 52 can be allowed to be configured to visually present the physiological state signal S2. When the information providing module 5 can be allowed to be configured as at least one or more sound players 53 for playing the physiological state signal S2, the sound player 53 can be allowed to be configured to audibly present the physiological state signal S2. When the information providing module 5 can be allowed to be configured as at least one or more vibration generators 54 for generating different vibration frequencies (or vibrations with different frequencies) based on changes in the physiological state signal S2, the vibration generator 54 can be configured to tangibly present the physiological state signal S2. However, the aforementioned details are disclosed for exemplary purposes only.

### [Second Embodiment]

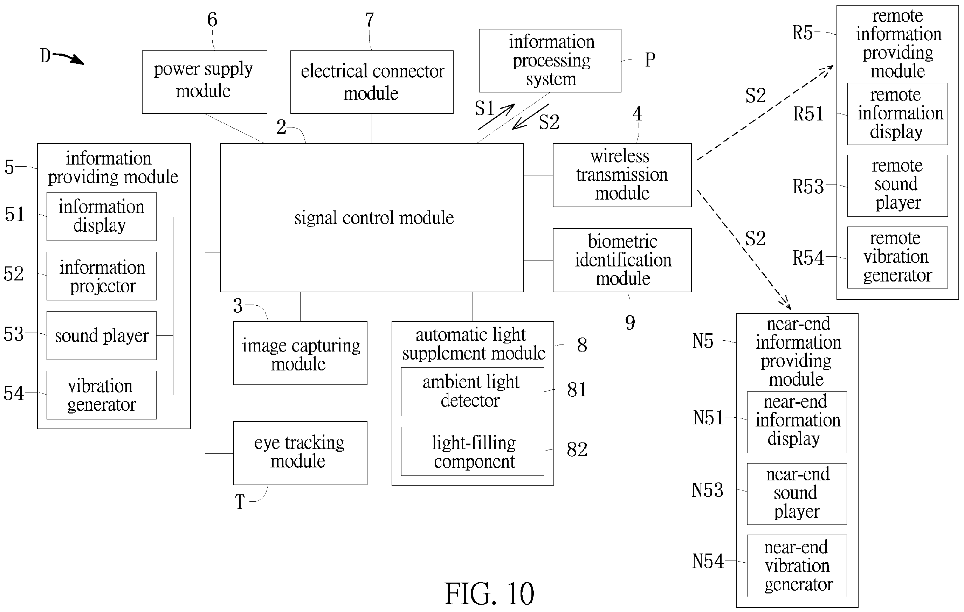

Referring to FIG. 2 to FIG. 10, a second embodiment of the present invention provides a physiological state detection device D (or a physiological and mental state detection device, or a physiological information detection device, or a physiological and mental information detection device) that can be applied to an electronic device (such as a pair of eyeglasses G, a helmet H or an eye mask E), which includes a device casing module (such as an eyeglass structure module 1A, a helmet structure module 1B, an eye mask structure module 1C), a signal control module 2, an image capturing module 3, a wireless transmission module 4, an information providing module 5 and a power supply module 6. Comparing FIG. 10 with FIG. 1, the main difference between the second embodiment and the first embodiment is as follows: in the second embodiment, the information processing system P can be directly installed inside the electronic device, and the physiological state detection device D can be electrically connected to the information processing system P in a wired manner (that is to say, the physiological state detection device D and the information processing system P can communicate with each other in a wired manner). For example, when the image capturing module 3 can be configured to obtain a plurality of eye image signals S 1 (or signals of eye images and characteristic images around the eyes) respectively corresponding to a plurality of eye images M (or facial images) of the user U through the control of the signal control module 2, the physiological state detection device D can be electrically connected to the information processing system P in a wired manner, so that the eye image signals S1 can be transmitted to an information processing system P in a wired manner for processing in order to obtain a physiological state signal S2 corresponding to the eye image signals S1, and the physiological state signal S2 that has been processed by the information processing system P can be transmitted back to the physiological state detection device D in a wired manner. It should be noted that the physiological state signal S2 that has been processed by the information processing system P can be wirelessly transmitted to a remote information providing module R5 (such as a portable electronic device, e.g., a mobile phone, a tablet or a laptop, or such as a non-portable electronic device, e.g., a desktop computer or a large system computer) away from the user U through the wireless transmission module 4. However, the aforementioned details are disclosed for exemplary purposes only.

### [Beneficial Effects of the Embodiments]

In conclusion, in the physiological state detection device D and the electronic device provided by the present invention, by virtue of "the image capturing module 3 being electrically connected to the signal control module 2" and "the information providing module 5 being disposed inside the device casing module and electrically connected to the signal control module 2," when the image capturing module 3 is optionally configured to be used, the image capturing module 3 can be allowed to be configured through the signal control module 2 to continuously or discontinuously capture a plurality of eye images M (each eye image includes at least one scleral image M1 with microvascular characteristics or at least one eyelid image M2 with microvascular characteristics) or a plurality of facial images (each facial image includes at least one scleral image M1 with microvascular characteristics or at least one eyelid image M2 with microvascular characteristics) of a user U, and when the information providing module 5 is optionally configured to be used, the information providing module 5 can be allowed to be configured through the signal control module 2 to present a physiological state signal S2 corresponding to the eye images M for reference by relevant personnel.

Furthermore, in the physiological state detection method provided by the present invention, by virtue of "providing an electronic device equipped with a physiological state detection device D," "identifying an identity of a user U using the electronic device through a biometric module 9 of the physiological state detection device D," "continuously or discontinuously capturing a plurality of eye images M of the user U within a predetermined period by an image capturing module 3 of the physiological state detection device D, thereby obtaining blood flow changes or spectral changes in/of capillaries of scleras or eyelids of the user U" and "processing the eye images M by an information processing system P, thereby obtaining a physiological state signal S2 corresponding to the eye images M," the physiological state signal S2 can be presented by an information providing module 5 of the physiological state detection device D for reference by relevant personnel. For example, when the information providing module 5 can be allowed to be configured as an information display 51 for displaying the physiological state signal S2, the information display 51 can be allowed to be configured to visually present the physiological state signal S2 for reference by relevant personnel. When the information providing module 5 can be allowed to be configured as an information projector 52 for projecting the physiological state signal S2 to at least one eye based on eyeball position information captured by an eye tracking module T, the information projector 52 can be allowed to be configured to visually present the physiological state signal S2 for reference by relevant personnel. When the information providing module 5 can be allowed to be configured as a sound player 53 for playing the physiological state signal S2, the sound player 53 can be allowed to be configured to audibly present the physiological state signal S2 for reference by relevant personnel. When the information providing module 5 can be allowed to be configured as a vibration generator 54 for generating different vibration frequencies based on changes in/of the physiological state signal S2, the vibration generator 54 can be configured to tangibly present the physiological state signal S2 for reference by relevant personnel.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its spirit and scope.

## Claims

1. A physiological state detection device (D) applied to an electronic device, **characterized by** comprising:
a device casing module;
a signal control module (2) disposed inside the device casing module;
an image capturing module (3) electrically connected to the signal control module (2);
a wireless transmission module (4) disposed inside the device casing module and electrically connected to the signal control module (2);
an information providing module (5) disposed inside the device casing module and electrically connected to the signal control module (2); and
a power supply module (6) disposed inside the device casing module and electrically connected to the signal control module (2);
wherein, when the image capturing module (3) is optionally configured to be used, the image capturing module (3) is allowed to be configured through the signal control module (2) to continuously or discontinuously capture a plurality of eye images (M) of a user (U) using the electronic device within a predetermined period, thereby obtaining a plurality of eye image signals (S1) respectively corresponding to the eye images (M) of the user (U);
wherein, when the wireless transmission module (4) is optionally configured to be used, the wireless transmission module (4) is allowed to be configured through the signal control module (2) to transmit the eye image signals (S 1) to an information processing system (P), thereby obtaining a physiological state signal (S2) corresponding to the eye image signals (S 1);
wherein, when the wireless transmission module (4) is optionally configured to be used, the wireless transmission module (4) is allowed to be configured through the signal control module (2) to receive the physiological state signal (S2) that is obtained through processing by the information processing system (P);
wherein, when the information providing module (5) is optionally configured to be used, the information providing module (5) is allowed to be configured through the signal control module (2) to present the physiological state signal (S2) for reference by relevant personnel;
wherein, when the power supply module (6) is optionally configured to be used, the power supply module (6) is allowed to be configured through the signal control module (2) to supply power to the signal control module (2), the image capturing module (3), the wireless transmission module (4) and the information providing module (5);
wherein each of the eye images (M) of the user (U) includes at least one scleral image (M1) with microvascular characteristics or at least one eyelid image (M2) with microvascular characteristics.

2. The physiological state detection device (D) according to claim 1,
wherein the physiological state detection device (D) further comprises an electrical connector module (7) disposed inside the device casing module and electrically connected to the signal control module (2);
wherein, when the electrical connector module (7) is optionally configured to be used, the electrical connector module (7) is allowed to be configured through the signal control module (2) to communicate with an external system in a wired manner;
wherein the physiological state detection device (D) further comprises an automatic light supplement module (8) disposed inside the device casing module and electrically connected to the signal control module (2), and the automatic light supplement module (8) includes an ambient light detector (81) and a light-filling component (82);
wherein, when the ambient light detector (81) is optionally configured to be used, the ambient light detector (81) is allowed to be configured through the signal control module (2) to detect an ambient light around the user (U), thereby obtaining ambient light information;
wherein, when the light-filling component (82) is optionally configured to be used, the light-filling component (82) is allowed to be configured through the signal control module (2) to determine whether to provide a predetermined invisible light to the user (U) based on whether the ambient light information meets a predetermined requirement;
wherein the physiological state detection device (D) further comprises a biometric module (9) disposed inside the device casing module and electrically connected to the signal control module (2), and the biometric module (9) is configured as an iris recognition module, a sclera recognition module, a palmprint recognition module, a fingerprint recognition module or a facial recognition module;
wherein, when the biometric module (9) is configured as the iris recognition module or the sclera recognition module, the iris recognition module or the sclera recognition module is allowed to be configured through the signal control module (2) to capture at least one iris image or sclera image of the user (U), thereby identifying whether the user (U) is qualified to use the physiological state detection device (D);
wherein, when the biometric module (9) is configured as the fingerprint recognition module or the palmprint recognition module, the fingerprint recognition module or the palmprint recognition module is allowed to be configured through the signal control module (2) to capture at least one fingerprint image or palmprint image of the user (U), thereby identifying whether the user (U) is qualified to use the physiological state detection device (D);
wherein, when the biometric module (9) is configured as the facial recognition module, the facial recognition module is allowed to be configured through the signal control module (2) to capture at least one facial image of the user (U), thereby identifying whether the user (U) is qualified to use the physiological state detection device (D).

3. The physiological state detection device (D) according to claim 1,
wherein the image capturing module (3) includes at least one lower pixel facial image capturer and at least one higher pixel eye image capturer, and the at least one lower pixel facial image capturer is configured to confirm an eye position range of the user (U) to obtain eye position information, and the at least one higher pixel eye image capturer is configured to quickly and accurately capture the at least one scleral image (M1) or the at least one eyelid image (M2) of the user (U) based on the eye position information provided by the at least one lower pixel facial image capturer;
wherein the image capturing module (3) includes an angle-adjustable lens structure, and the angle-adjustable lens structure is configured to manually or automatically adjust an image capturing angle relative to the user (U);
wherein, when the image capturing module (3) is optionally configured to be used, the image capturing module (3) is allowed to be configured through the signal control module (2) to manually or automatically adjust the image capturing angle of the angle-adjustable lens structure relative to the user (U), until the facial image of the user (U) is completely captured by the image capturing module (3);
wherein the physiological state signal (S2) includes heart rate, blood pressure, blood oxygen, lactate, blood sugar, sleepiness and alcohol concentration;
wherein the number of the eye images (M) obtained by the image capturing module (3) within the predetermined period exceeds a predetermined value, thereby obtaining blood flow changes or spectral changes in capillaries of the user's scleras or eyelids;
wherein the electronic device is a head-mounted device (G, H, E), a wearable device, a desktop computer, a portable computer, a card reader of an access control system, or a transportation.

4. The physiological state detection device (D) according to claim 1,
wherein, when the information providing module (5) is optionally configured to be used, the information providing module (5) is allowed to be configured as an information display (51) for displaying the physiological state signal (S2), so that the information display (51) is allowed to be configured to visually present the physiological state signal (S2);
wherein, when the wireless transmission module (4) is optionally configured to be used, the wireless transmission module (4) is allowed to be configured through the signal control module (2) to transmit the physiological state signal (S2) to a near-end information providing module (N5) adjacent to the user (U);
wherein the physiological state detection device (D) is configured to perform information acquisition operation, information transmission operation, and information calculation operation through the near-end information providing module (N5);
wherein, when the near-end information providing module (N5) is optionally configured to be used, the near-end information providing module (N5) is allowed to be configured as a near-end information display (N51) for displaying the physiological state signal (S2), so that the near-end information display (N51) is allowed to be configured to visually present the physiological state signal (S2);
wherein, when the information processing system (P) is configured to transmit the physiological state signal (S2) to a remote information providing module (R5) away from the user (U), the remote information providing module (R5) is allowed to be configured as a remote information display (R51) for displaying the physiological state signal (S2), so that the remote information display (R51) is allowed to be configured to visually present the physiological state signal (S2).

5. The physiological state detection device (D) according to claim 1,
wherein, when the information providing module (5) is optionally configured to be used, the information providing module (5) is allowed to be configured as an information projector (52) for projecting the physiological state signal (S2) to at least one eye based on eyeball position information captured by an eye tracking module (T), so that the information projector (52) is allowed to be configured to visually present the physiological state signal (S2);
wherein, when the wireless transmission module (4) is optionally configured to be used, the wireless transmission module (4) is allowed to be configured through the signal control module (2) to transmit the physiological state signal (S2) to a near-end information providing module (N5) adjacent to the user (U);
wherein the physiological state detection device (D) is configured to perform information acquisition operation, information transmission operation, and information calculation operation through the near-end information providing module (N5).

6. The physiological state detection device (D) according to claim 1,
wherein, when the information providing module (5) is optionally configured to be used, the information providing module (5) is allowed to be configured as a sound player (53) for playing the physiological state signal (S2), so that the sound player (53) is allowed to be configured to audibly present the physiological state signal (S2);
wherein, when the wireless transmission module (4) is optionally configured to be used, the wireless transmission module (4) is allowed to be configured through the signal control module (2) to transmit the physiological state signal (S2) to a near-end information providing module (N5) adjacent to the user (U);
wherein the physiological state detection device (D) is configured to perform information acquisition operation, information transmission operation, and information calculation operation through the near-end information providing module (N5);
wherein, when the near-end information providing module (N5) is optionally configured to be used, the near-end information providing module (N5) is allowed to be configured as a near-end sound player (N53) for playing the physiological state signal (S2), so that the near-end sound player (N53) is allowed to be configured to audibly present the physiological state signal (S2);
wherein, when the information processing system (P) is configured to transmit the physiological state signal (S2) to a remote information providing module (R5) away from the user (U), the remote information providing module (R5) is allowed to be configured as a remote sound player (R53) for playing the physiological state signal (S2), so that the remote sound player (R53) is allowed to be configured to audibly present the physiological state signal (S2).

7. The physiological state detection device (D) according to claim 1,
wherein, when the information providing module (5) is optionally configured to be used, the information providing module (5) is allowed to be configured as a vibration generator (54) for generating different vibration frequencies based on changes in the physiological state signal (S2), so that the vibration generator (54) is configured to tangibly present the physiological state signal (S2);
wherein, when the wireless transmission module (4) is optionally configured to be used, the wireless transmission module (4) is allowed to be configured through the signal control module (2) to transmit the physiological state signal (S2) to a near-end information providing module (N5) adjacent to the user (U);
wherein the physiological state detection device (D) is configured to perform information acquisition operation, information transmission operation, and information calculation operation through the near-end information providing module (N5);
wherein, when the near-end information providing module (N5) is optionally configured to be used, the near-end information providing module (N5) is allowed to be configured as a near-end vibration generator (N54) for generating different vibration frequencies based on changes in the physiological state signal (S2), so that the near-end vibration generator (N54) is allowed to be configured to tangibly present the physiological state signal (S2);
wherein, when the information processing system (P) is configured to transmit the physiological state signal (S2) to a remote information providing module (R5) away from the user (U), the remote information providing module (R5) is allowed to be configured as a remote vibration generator (R54) for generating different vibration frequencies based on changes in the physiological state signal (S2), so that the remote vibration generator (R54) is allowed to be configured to tangibly present the physiological state signal (S2).

8. An electronic device configured for using a physiological state detection device (D), **characterized by** comprising:
a device casing module;
a signal control module (2) disposed inside the device casing module;
an image capturing module (3) electrically connected to the signal control module (2); and
an information providing module (5) disposed inside the device casing module and electrically connected to the signal control module (2);
wherein, when the image capturing module (3) is optionally configured to be used, the image capturing module (3) is allowed to be configured through the signal control module (2) to continuously or discontinuously capture a plurality of eye images (M) of a user (U) within a predetermined period;
wherein, when the information providing module (5) is optionally configured to be used, the information providing module (5) is allowed to be configured through the signal control module (2) to present a physiological state signal (S2) corresponding to the eye images (M);
wherein each of the eye images (M) of the user (U) includes at least one scleral image (M1) with microvascular characteristics or at least one eyelid image (M2) with microvascular characteristics.

9. The electronic device configured for using the physiological state detection device (D) according to claim 8,
wherein the physiological state detection device (D) further comprises an electrical connector module (7) disposed inside the device casing module and electrically connected to the signal control module (2);
wherein, when the electrical connector module (7) is optionally configured to be used, the electrical connector module (7) is allowed to be configured through the signal control module (2) to communicate with an external system in a wired manner;
wherein the physiological state detection device (D) further comprises an automatic light supplement module (8) disposed inside the device casing module and electrically connected to the signal control module (2), and the automatic light supplement module (8) includes an ambient light detector (81) and a light-filling component (82);
wherein, when the ambient light detector (81) is optionally configured to be used, the ambient light detector (81) is allowed to be configured through the signal control module (2) to detect an ambient light around the user (U), thereby obtaining ambient light information;
wherein, when the light-filling component (82) is optionally configured to be used, the light-filling component (82) is allowed to be configured through the signal control module (2) to determine whether to provide a predetermined invisible light to the user (U) based on whether the ambient light information meets a predetermined requirement;
wherein the physiological state detection device (D) further comprises a biometric module (9) disposed inside the device casing module and electrically connected to the signal control module (2), and the biometric module (9) is configured as an iris recognition module, a sclera recognition module, a palmprint recognition module, a fingerprint recognition module or a facial recognition module;
wherein, when the biometric module (9) is configured as the iris recognition module or the sclera recognition module, the iris recognition module or the sclera recognition module is allowed to be configured through the signal control module (2) to capture at least one iris image or sclera image of the user (U), thereby identifying whether the user (U) is qualified to use the physiological state detection device (D);
wherein, when the biometric module (9) is configured as the fingerprint recognition module or the palmprint recognition module, the fingerprint recognition module or the palmprint recognition module is allowed to be configured through the signal control module (2) to capture at least one fingerprint image or palmprint image of the user (U), thereby identifying whether the user (U) is qualified to use the physiological state detection device (D);
wherein, when the biometric module (9) is configured as the facial recognition module, the facial recognition module is allowed to be configured through the signal control module (2) to capture at least one facial image of the user (U), thereby identifying whether the user (U) is qualified to use the physiological state detection device (D);
wherein the physiological state signal (S2) includes heart rate, blood pressure, blood oxygen, lactate, blood sugar, sleepiness and alcohol concentration;
wherein the number of the eye images (M) obtained by the image capturing module (3) within the predetermined period exceeds a predetermined value, thereby obtaining blood flow changes or spectral changes in capillaries of the user's scleras or eyelids;
wherein, when the information providing module (5) is allowed to be configured as an information display (51) for displaying the physiological state signal (S2), the information display (51) is allowed to be configured to visually present the physiological state signal (S2);
wherein, when the information providing module (5) is allowed to be configured as an information projector (52) for projecting the physiological state signal (S2) to at least one eye based on eyeball position information captured by an eye tracking module (T), the information projector (52) is allowed to be configured to visually present the physiological state signal (S2);
wherein, when the information providing module (5) is allowed to be configured as a sound player (53) for playing the physiological state signal (S2), the sound player (53) is allowed to be configured to audibly present the physiological state signal (S2);
wherein, when the information providing module (5) is allowed to be configured as a vibration generator (54) for generating different vibration frequencies based on changes in the physiological state signal (S2), the vibration generator (54) is configured to tangibly present the physiological state signal (S2);
wherein when the physiological state detection device (D) is allowed to be configured to be electrically connected to an information processing system (P), the information processing system (P) is allowed to be configured to process the eye images (M), thereby obtaining the physiological state signal (S2) corresponding to the eye images (M);
wherein, when the image capturing module (3) is optionally configured to be used, the image capturing module (3) is allowed to be configured through the signal control module (2) to continuously or discontinuously capture a plurality of facial images of the user (U) using the electronic device within a predetermined period, thereby obtaining a plurality of eye image signals (S 1) respectively corresponding to the facial images of the user (U);
wherein the number of the facial images obtained by the image capturing module (3) within the predetermined period exceeds a predetermined value, thereby obtaining blood flow changes or spectral changes in capillaries of the user's scleras or eyelids.

10. A physiological state detection method, **characterized by** comprising:
providing an electronic device equipped with a physiological state detection device (D);
identifying an identity of a user (U) using the electronic device through a biometric module (9) of the physiological state detection device (D);
continuously or discontinuously capturing a plurality of eye images (M) of the user (U) within a predetermined period by an image capturing module (3) of the physiological state detection device (D), thereby obtaining blood flow changes or spectral changes in capillaries of the user's scleras or eyelids;
processing the eye images (M) by an information processing system (P), thereby obtaining a physiological state signal (S2) corresponding to the eye images (M); and
presenting the physiological state signal (S2) by an information providing module (5) of the physiological state detection device (D) for reference by relevant personnel;
wherein, when the information providing module (5) is allowed to be configured as an information display (51) for displaying the physiological state signal (S2), the information display (51) is allowed to be configured to visually present the physiological state signal (S2);
wherein, when the information providing module (5) is allowed to be configured as an information projector (52) for projecting the physiological state signal (S2) to at least one eye based on eyeball position information captured by an eye tracking module (T), the information projector (52) is allowed to be configured to visually present the physiological state signal (S2);
wherein, when the information providing module (5) is allowed to be configured as a sound player (53) for playing the physiological state signal (S2), the sound player (53) is allowed to be configured to audibly present the physiological state signal (S2);
wherein, when the information providing module (5) is allowed to be configured as a vibration generator (54) for generating different vibration frequencies based on changes in the physiological state signal (S2), the vibration generator (54) is configured to tangibly present the physiological state signal (S2).
